# EUROPEAN PATENT SPECIFICATION

(11) **EP 1 904 377 B1**
(45) Date of publication and mention of the grant of the patent: **21.08.2013**
(21) Application number: 06788013.8
(22) Date of filing: 20.07.2006
(51) Int. Cl.: A61B 19/00, A61B 19/02, B01L 1/00, B65D 25/24, B65F 1/00, B65F 1/06

(54) **IN-WALL WASTE RECEPTACLES FOR HOSPITAL AND LABORATORY ENVIRONMENTS**
WANDINTERNE ABFALLBEHÄLTER FÜR KRANKENHÄUSER UND LABORS
RÉCIPIENTS À DÉCHETS POUR HOPITAUX ET LABORATOIRES

(30) Priority: 20.07.2005 US 701106 P
(43) Date of publication of application: 02.04.2008
(73) Proprietor: Optimus Licensing AG, 6300 Zug (CH)
(72) Inventor: MANGIARDI, John, R., Greenwich, CT 06830 (US)
(74) Representative: Manitz, Gerhart
(86) International application number: PCT/US2006/028234
(87) International publication number: WO 2007/012045

(56) References cited:
- FR-A1- 2 838 410
- GB-A- 191 121 247
- US-A- 1 708 895
- US-A- 2 445 914
- US-A- 3 211 367
- US-A- 3 211 367
- US-A- 4 925 048
- US-A- 5 259 501
- US-A- 5 740 923

## Description

### BACKGROUND OF THE INVENTION - FIELD OF INVENTION

The present invention relates to a device and method for disposing of waste in medical or laboratory environments. US 3 211 367 A discloses the features of the preamble of claim 1. US 5 740 923 A and FR 2 838 410 A1 disclose receptacles to be mounted in a vertical position.

### BACKGROUND OF THE INVENTION

When waste is generated in the medical or scientific environment, it consumes valuable space. Typically, waste generated during a medical procedure or a scientific experiment is disposed in specially marked waste receptacles that exist as stand-alone containers such as the foot-maneuvered bucket of Noack in U.S. Pat. No. 4,925,048. For each type of waste, a different receptacle is usually required such as for sharps, regular trash, and biologics. An invention that can provide a disposal method that does not eliminate valuable space in the working environment would be of benefit. Further, medical or scientific environments typically require strictly sterile working conditions. A device that can incorporate a means for maintaining sterility in its design would be of further benefit.

It is therefore an object of this invention to provide a sterile, disposable receptacle that does not eliminate space in the working environment.

It is another object of this invention to provide a receptacle that is lightweight and cost-effective.

It is yet another object of this invention to integrate said receptacle with existing disposal means such as hospital biologic waste bags.

At least one of the above objects is met in whole or in part by the invention and additional objects are shown by the following description and claims.

### SUMMARY OF THE INVENTION

A trash receptacle is described. It comprises a molded, lightweight, plastic tube, closed at its bottom end, and adapted to fit within an opening in a wall. The tube has an opening that can receive a bag, said bag capable of being held in place by the tube after insertion of the tube into a specially adapted opening in the wall, said opening in the wall also comprised as part of the present invention. The wall is specially adapted to be capable of holding said tube by the weight of said tube alone. The tube is shaped such that after insertion of the tube into the opening in the wall, the center of gravity of the tube is adjacent to, as opposed to comprised somewhere within the tube, thereby helping maintain the tube stably within the opening in the wall. The tube is further adapted to allow a variety of plugs to fit within the opening of the tube to allow closure of the receptacle should disposal of the tube be necessary.

In a typical use, the empty tube has a bag inserted into the opening within the tube; the upper portion of the bag may be wrapped around the opening of the tube to help secure the bag. An O-ring or other sealing device, adapted to snap over the waste bag and onto the cartridge, may then be secured over the bag and cartridge. The O-ring or other sealing device is preferably color-coded and can snap over the bag and cartridge in a variety of manners. In one manner, the O-ring has a flexible hook which secures over the lip of the cartridge, or, alternatively, the O-ring may be fashioned like a standard kitchen container lid (like those manufactured by Tupperware®). In any embodiment of the O-ring, the cartridge is likewise adapted to secure to the O-ring. Other sealing devices, similar to O-rings but not necessarily shaped as an "O", may also be used, such as a square sealing device.

The tube is then inserted into the wall opening. After the bag is full, the bag may be removed and a new bag inserted. The bag maintains the sterility of the cartridge and hence helps maintain the sterility of the environment. If a bag breaks, the tube may be closed with a plug and disposed of. Since the tube is made of lightweight plastic, disposal is easy and cost-conscious. The receptacles may also be fitted with special sharps plugs to allow disposal of sharp instruments such as needles or scapula tips. The special sharp plugs are adapted to prevent a person from being pricked or cut by the contents of the cartridge. In a preferred embodiment, the sharps plug snaps into the tube and after the tube is full, the sharps plug may be sealed by a plastic sheet that slides over the plugs opening and snaps permanently into place. Lastly, the wall openings may be arrayed on the wall to provide convenient access to a variety of disposal tubes. It is preferred that the wall openings, the O-ring snaps or other snapable securing devices, or all be color-coded for easy identification of the type of waste receptacle provided by the wall opening and tube combination.

### BRIEF DESCRIPTION OF THE DRAWINGS

The present invention can best be understood in connection with the accompanying drawings. It is noted that the invention is not limited to the precise embodiments shown in drawings, in which:
- Fig. 1 is a perspective view of the tube showing the opening and one possible shape of the tube.
- Fig. 2 is a perspective view of one possible embodiment of a sharps plug.
- Fig. 3 is a perspective view of one possible embodiment of a waste tube plug.
- Fig. 4 is a side-view detail of the tube being inserted into the wall with a waste bag inserted into the tube.
- Fig. 5 is a direct onward view of one possible array of waste receptacles including a bottom sharps receptacle.
- Fig. 6 is a side-view detail of the tube being inserted into the wall with a waste bag inserted into the tube and with the waste bag held into place by a snapable securing device.

### BRIEF DESCRIPTION OF REFERENCE NUMERALS

100 Tube Member Opening; 102 Tube Member Lip; 104 Tube Member Molded Surface; 106 Tube Member/Cartridge; 120 Sharps Closing Sheet; 122 Sharps Plug Opening; 124 Sharps Plug Surface; 126 Sharps Plug Protective House; 128 Sharps Plug; 130 Waste Plug; 132 Waste Plug Knob; 150 Wall Adapted For Tube Member; 154 Center Of Gravity Of Tube Member; 156 Waste Bag; 160 Wall Chamfer; 170 Trash Receptacle Array; 172 Trash Receptacles; 174 Biologics Receptacle; 176 Sharps Receptacle; 178 Color Coding Means; 200 Snapable Sealer; 202 Sealer Perimeter; 204 Sealer Snap

### DETAILED DESCRIPTION OF THE INVENTION

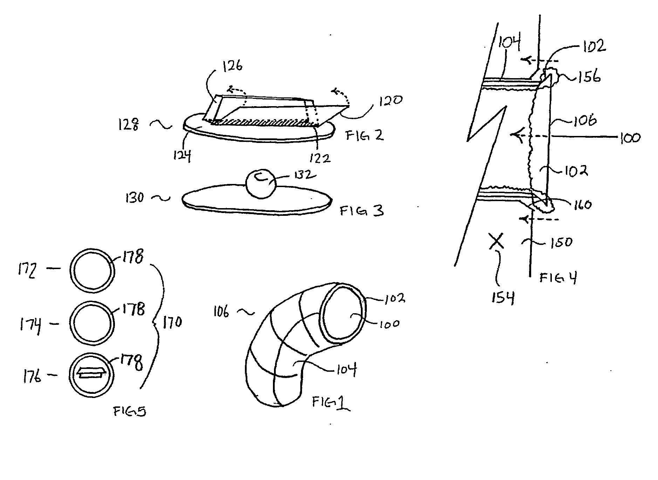

As seen in Fig. 1, the waste receptacle cartridge 106 has a preferred shape similar to a rounded upside-down "L". Other shapes may used, provided they help hold the cartridge into wall. For example, a non-upside-down "L" shape would not be appropriate.

The outside molded surface can be a variety of textures but is preferably composed, as is the entirety of the cartridge **106,** out of lightweight plastic. The shaping and texture may be adapted, as it is in the preferred embodiment, to allow a smooth sliding action into or out of the second wall-element. Alternatively, the texture may be adjusted to make movement more resistant.

The tube structure of **106** defines an interior **100.** As can be seen, tube **106** has a bottom surface providing a tube with a single entrance. The outside perimeter of lip **102** is designed to fit snugly within the second wall-element and to allow a variety of plugs to fit inside the perimeter of the lip.

In Fig. 2, a sharps plug **128** is shown. The surface of the plug **124** terminates at the outside circumference that is adapted to fit inside the lip **102** of the cartridge/tube **106.** At the center of the plug **124** is an opening **122** into the interior **100** of cartridge **106.** The opening **122** is surrounded by house **126** that prevents accidental insertion of a user digit into the opening. Sheet **120** may close into the interior of house **126,** thereby closing hole **122.** House **126** is adapted to prevent **120** from reopening the hole at **122** by, for example, ridges adapted to allow one-way travel of **120** only (ridges not shown).

In Fig. 3, a typical embodiment of a cartridge plug **130** is shown. In a typical use, the interior waste bag **156** (not shown) inside cavity **100** of cartridge **106** is removed. A new, empty waste bag may then be inserted. If the bag is broken, plug **130** may be inserted snugly into the opening **100** and sealing around lip **102** using knob **132** as a handle.

Fig. 4 displays a side-view of the cartridge **106** and wall opening **150** combination with cartridge **106** and its surface **104** nearly fully inserted into the wall-opening. Cartridge **106** has a waste bag **156** inserted into cavity **100** and wrapped around the lip **102.** As the cartridge is fully inserted into the wall opening, bag **156** is pressed against the outside of lip **102** and the chamfer **160.** The chamfer/lip design is one typical embodiment. The chamfer can instead be a recessed notch with the lip adapted to join with said notch. As can be seen by the mark "X", the center of gravity in all preferred embodiments is disposed below the entrance to cavity **100** and near the wall. This helps cartridge **106** maintain its position inside the wall cavity of **150** under its own weight.

Fig. 5 displays a possible arrangement of an array of waste receptacles having a rounded entrance. For example, waste receptacles **172, 174,** and **176** may be trash, biologics, and sharps respectively, and may be further have color-coding means **178,** such as by a colored sealing snap, a color lip perimeter, or a colored cartridge, thereby forming trash array **170.** Color-coding works to identify the type of waste to be deposited in a cartridge; for example, a red sealing snap such as a red O-ring could be used to identify a cartridge as accepting biological waste, whereas a blue colored sealing snap could indicate regular trash waste.

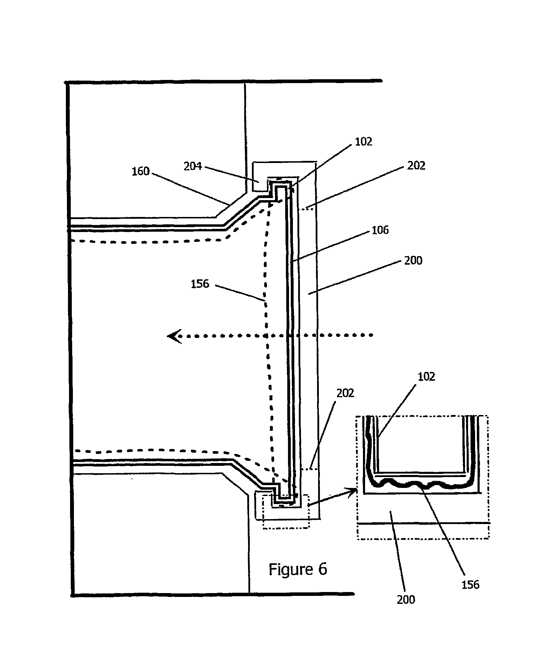

Fig. 6 displays an additional embodiment of the invention. With respect to the drawing, tube **106** is seen with bag **156** inserted, and tube and bag inserted into the wall opening resting against **160.** The lip **102** in this embodiment is adapted to allow sealing device **200** to snap onto the lip **102** by snap **204.** Alternatively, it is conceived that the sealing device will snap onto lip **102** as would a standard kitchen container lid such as those manufactured by Tupperware®. The above sharps and seal plugs may also be adapted with snaps to be held into place about the cartridge. Lines **202** demark the inner perimeter of the sealing device, which is (inside of the lines) open to allow access to the cartridge. Sealing device **200** functions to securely hold bag **156.** The sealing devices are preferably color-coded to allow identification of the type of waste to be disposed of in the attached waste bag and or cartridge.

In the foregoing description, certain terms and visual depictions are used to illustrate the preferred embodiment. However, no unnecessary limitations are to be construed by the terms used or illustrations depicted, beyond what is shown in the prior art, since the terms and illustrations are exemplary only, and are not meant to limit the scope of the present invention.

It is further known that other modifications may be made to the present invention, without departing the scope of the invention, as noted in the appended claims.

## Claims

1. A trash receptacle, comprising
a cartridge (106) which is adapted to be received and inserted into an opening (150) in a wall,
said cartridge (106) having a cavity (100) and an opening to said cavity (100),
said cartridge (106) adapted to seal against said wall,
**characterized in that**
said trash receptacle is for hospital or laboratory environment and said wall is in the hospital or laboratory environment,
said cartridge (106) has a center of gravity (X) not comprised on or within said cartridge (106), and
said cartridge (106) is capable of removal after insertion into said opening (150).

2. The trash receptacle of claim 1 further comprising a waste bag (156) inside of said cavity (100), an upper portion of said waste bag (156) optionally wrapped around the perimeter to said opening.

3. The trash receptacle of claim 1 further comprising a plug (128) adapted to seal said opening.

4. The trash receptacle of claim 3 in which said plug (128) is further adapted with a grip comprised on an outer surface (124) of said plug (128).

5. The trash receptacle of claim 3 in which said plug (128) is further adapted to provide a channel (122) from a top of said plug (128) to a bottom of said plug (128).

6. The trash receptacle of claim 5 in which said channel (122) in said plug (128) is surrounded by a protective house (126) adapted to prevent accidental insertion of a user's digit and in which said channel (122) in said plug (128) may be sealed by a sheet (120) that permanently shuts said channel (122).

7. The trash receptacle of claim 3 in which said plug (128) is color-coded.

8. The trash receptacle of claim 1 in which the perimeter to said opening (122) is color-coded to indicate the type of waste to be inserted into said cartridge's cavity (100).

9. The trash receptacle of claim 1 in which the perimeter to said opening comprises a recessed notch adapted to allow a user digit to be inserted thereby allowing said user digit to remove said cartridge (106).

10. The trash receptacle of claim 1 further comprising a sealing device (200), which snaps over a waste bag (156) and onto a lip (102) of said cartridge (106), said lip (102) on the perimeter of said opening.

11. The trash receptacle of claim 10 in which said sealing device (200) is color-coded.

12. The trash receptacle of claim 11 in which said sealing device (200) is an O-ring.

13. A method of sanitary disposal of hospital or laboratory waste comprising
providing a trash receptacle as described in claim 11;
inserting a waste bag (156) into said cartridge (106);
inserting said cartridge (106) into said opening (150);
disposing of waste into said cartridge (106) by placing said waste into said waste bag (156) inside said cartridge (106);
sealing said cartridge (106) with a plug (130) adapted to seal said cartridge (106) entrance if said waste bag (156) is broken, removing said cartridge (106) from said opening, inserting a new waste bag (156) into a new cartridge (106), and then inserting said new cartridge (106) into said opening (150);
removing said waste bag (156) if full and unbroken and replacing said waste bag (156) with a new waste bag (156).

14. The method of claim 13 further comprising the step of placing said color-coded sealing device (200) over said waste bag (156) after said waste bag (156) is inserted into said cartridge (106) and then snapping said sealing device (200) onto said cartridge (106) and also comprising the step of removing said color-coded sealing device (200) before sealing of said cartridge (106) or before removing of said waste bag (156).

15. A method of sanitary disposal of hospital or laboratory waste comprising
providing a trash receptacle as described in claim 6 in which said plug (128) is color-coded;
inserting said cartridge (106) into said opening (150);
disposing of waste into said cartridge (106) by placing said waste through said channel (122) in said plug (128) into said cartridge cavity (100);
sealing said channel (122) with said sheet (120) when said cartridge cavity (100) is full;
removing said cartridge (106) from said opening;
replacing said cartridge (106) and plug (128) combination with a new cartridge (106) and plug (128) combination.

## Patentansprüche

1. Abfallbehältnis, umfassend:
eine Kartusche (106), die zur Aufnahme und zum Einsetzen in eine Öffnung (150) in einer Wand angepasst ist,
wobei die Kartusche (106) einen Hohlraum (100) und eine Öffnung zu dem Hohlraum (100) besitzt,
wobei die Kartusche (100) zur Abdichtung gegen die Wand angepasst ist,
**dadurch gekennzeichnet, dass** das Abfallbehältnis für Krankenhaus- oder Laborumgebung dient und sich die Wand in der Krankenhaus- oder Laborumgebung befindet,
wobei die Kartusche (106) einen Schwerpunkt (X) aufweist, der nicht an oder in der Kartusche (106) enthalten ist, und
wobei die Kartusche (106) zur Entfernung nach einem Einsetzen in die Öffnung (150) in der Lage ist.

2. Abfallbehältnis nach Anspruch 1,
ferner mit einem Abfallbeutel (156) in dem Hohlraum (100), wobei ein oberer Abschnitt des Abfallbeutels (156) optional um den Umfang zu der Öffnung gewickelt wird.

3. Abfallbehältnis nach Anspruch 1,
ferner mit einem Verschluss (128), der zum Abdichten der Öffnung angepasst ist.

4. Abfallbehältnis nach Anspruch 3,
wobei der Verschluss (128) ferner mit einem Griff angepasst ist, der an einer Außenfläche (124) des Verschlusses (128) vorgesehen ist.

5. Abfallbehältnis nach Anspruch 3,
wobei der Verschluss (128) ferner derart angepasst ist, einen Kanal (122) von einem oberen Bereich des Verschlusses (128) zu einem unteren Bereich des Verschlusses (128) bereitzustellen.

6. Abfallbehältnis nach Anspruch 5,
wobei der Kanal (122) in dem Verschluss (128) durch ein Schutzgehäuse (126) umgeben ist, das derart angepasst ist, ein unabsichtliches Einführen eines Fingers eines Anwenders zu verhindern, und wobei der Kanal (122) in dem Verschluss (128) durch eine Lage (120) abgedichtet sein kann, die den Kanal (122) permanent verschließt.

7. Abfallbehältnis nach Anspruch 3,
wobei der Verschluss (128) farbcodiert ist.

8. Abfallbehältnis nach Anspruch 1,
wobei der Umfang zu der Öffnung (122) farbcodiert ist, um den Abfalltyp anzugeben, der in den Hohlraum der Kartusche (100) einzubringen ist.

9. Abfallbehältnis nach Anspruch 1,
wobei der Umfang zu der Öffnung eine ausgenommene Kerbe umfasst, die derart angepasst ist, zu ermöglichen, dass ein Finger eines Anwenders eingeführt werden kann, wodurch ermöglicht wird, dass der Finger des Anwenders die Kartusche (106) entfernen kann.

10. Abfallbehältnis nach Anspruch 1,
ferner mit einer Dichtungsvorrichtung (200), die über einen Abfallbeutel (156) und auf eine Lippe (102) der Kartusche (106) aufschnappt, wobei sich die Lippe (102) an dem Umfang der Öffnung befindet.

11. Abfallbehältnis nach Anspruch 10,
wobei die Dichtungsvorrichtung (200) farbcodiert ist.

12. Abfallbehältnis nach Anspruch 11,
wobei die Dichtungsvorrichtung (200) ein O-Ring ist.

13. Verfahren zur hygienischen Beseitigung von Krankenhaus- oder Laborabfall, umfassend:
Bereitstellen eines Abfallbehältnisses, wie in Anspruch 11 beschrieben ist;
Einsetzen eines Abfallbeutels (156) in die Kartusche (106);
Einsetzen der Kartusche (106) in die Öffnung (150);
Einbringen von Abfall in die Kartusche (106) durch Platzieren des Abfalls in dem Abfallbeutel (156) innerhalb der Kartusche (106);
Abdichten der Kartusche (106) mit einem Verschluss (130), der derart angepasst ist, den Eintritt der Kartusche (106) abzudichten,
wenn der Abfallbeutel (156) gebrochen ist; Entfernen der Kartusche (106) von der Öffnung, Einsetzen eines neuen Abfallbeutels (156) in eine neue Kartusche (106) und dann Einsetzen der neuen Kartusche (106) in die Öffnung (150);
Entfernen des Abfallbeutels (156), wenn er voll und unbeschadet ist,
und Ersetzen des Abfallbeutels (156) gegen einen neuen Abfallbeutel (156).

14. Verfahren nach Anspruch 13,
ferner mit dem Schritt zum Platzieren der farbcodierten Dichtungsvorrichtung (200) über dem Abfallbeutel (156), nachdem der Abfallbeutel (156) in die Kartusche (106) eingesetzt ist, und dann Aufschnappen der Dichtungsvorrichtung (200) auf die Kartusche (106) und auch mit dem Schritt zum Entfernen der farbcodierten Dichtungsvorrichtung (200) vor einem Abdichten der Kartusche (106) oder vor einem Entfernen des Abfallbeutels (156).

15. Verfahren zur hygienischen Beseitigung von Krankenhaus- oder Laborabfall, umfassend:
Bereitstellen eines Abfallbehältnisses, wie in Anspruch 6 beschrieben ist, bei dem der Verschluss (128) farbcodiert ist;
Einsetzen der Kartusche (106) in die Öffnung (150);
Einbringen von Abfall in die Kartusche (106) durch Platzieren des Abfalls durch den Kanal (122) in dem Verschluss (128) in den Kartuschenhohlraum (100);
Abdichten des Kanals (122) mit der Lage (120), wenn der Kartuschenhohlraum (100) voll ist;
Entfernen der Kartusche (106) von der Öffnung;
Ersetzen der Kombination aus Kartusche (106) und Verschluss (128) gegen eine neue Kombination aus Kartusche (106) und Verschluss (128).

## Revendications

1. Réceptacle à déchets, comprenant
une cartouche (106) qui est adaptée à être reçue et insérée dans une ouverture (150) dans une paroi,
ladite cartouche (106) ayant une cavité (100) et une ouverture vers ladite cavité (100),
ladite cartouche (106) étant adaptée à venir s'appliquer de manière étanche contre ladite paroi,
**caractérisé en ce que**
ledit réceptacle à déchets est destiné à un environnement en hôpital ou en laboratoire et ladite paroi se trouve dans l'environnement en hôpital ou en laboratoire,
ladite cartouche (106) a un centre de gravité (X) qui n'est pas compris sur ou à l'intérieur de ladite cartouche (106), et
ladite cartouche (106) est capable d'être enlevée après insertion dans ladite ouverture (150).

2. Réceptacle à déchets selon la revendication 1, comprenant en outre un sac à déchets (156) à l'intérieur de ladite cavité (100), une portion supérieure dudit sac à déchets (156) étant en option enveloppée autour du périmètre de ladite ouverture.

3. Réceptacle à déchets selon la revendication 1, comprenant en outre un bouchon (128) adapté à fermer ladite ouverture de manière étanche.

4. Réceptacle à déchets selon la revendication 3, dans lequel ledit bouchon (123) est en outre adapté avec un moyen d'agrippement compris sur une surface extérieure (124) dudit bouchon (128).

5. Réceptacle à déchets selon la revendication 3, dans lequel ledit bouchon (128) est en outre adapté à constituer un canal (122) depuis un sommet dudit bouchon (128) jusqu'à un fond dudit bouchon (128).

6. Réceptacle à déchets selon la revendication 5, dans lequel ledit canal (122) dans ledit bouchon (128) est entouré par un boîtier protecteur (126) adapté à empêcher une insertion accidentelle d'un doigt d'un utilisateur, et dans lequel ledit canal (122) dans ledit bouchon (128) peut être fermé de manière étanche par une feuille (128) qui ferme ledit canal (122) de manière permanente.

7. Réceptacle à déchets selon la revendication 3, dans lequel ledit bouchon (128) porte un code de couleur.

8. Réceptacle à déchets selon la revendication 1, dans lequel le périmètre de ladite ouverture (122) porte un code de couleur pour indiquer le type de déchets à introduire dans la cavité de ladite cartouche (100).

9. Réceptacle à déchets selon la revendication 1, dans lequel le périmètre de ladite ouverture comprend une encoche évidée adaptée à permettre d'introduire un doigt d'un utilisateur, permettant ainsi audit doigt de l'utilisateur d'enlever ladite cartouche (106).

10. Réceptacle à déchets selon la revendication 1, comprenant en outre un dispositif de scellement (200), qui coiffe un sac à déchets (156) et vient se prendre sur une lèvre (102) de ladite cartouche (106), ladite lèvre (102) se trouvant sur le périmètre de ladite ouverture.

11. Réceptacle à déchets selon la revendication 10, dans lequel ledit dispositif de scellement (200) porte un code de couleur.

12. Réceptacle à déchets selon la revendication 11, dans lequel ledit dispositif de scellement (200) est un joint torique.

13. Procédé pour rejeter de manière sanitaire des déchets en hôpital ou en laboratoire, comprenant les étapes consistant à
fournir un réceptacle à déchets tel que décrit dans la revendication 11 ; insérer un sac à déchets (156) dans ladite cartouche (106) ;
insérer ladite cartouche (106) dans ladite ouverture (150) ;
jeter des déchets dans ladite cartouche (106) en plaçant lesdits déchets dans ledit sac à déchets (156) à l'intérieur de ladite cartouche (106) ;
sceller ladite cartouche (106) avec un bouchon (130) adapté à fermer de manière étanche l'entrée de ladite cartouche (106) si ledit sac à déchets (156) est rompu, enlever ladite cartouche (106) depuis ladite ouverture, insérer un nouveau sac à déchets (156) dans une nouvelle cartouche (106), et ensuite insérer ladite nouvelle cartouche (106) dans ladite ouverture (150) ;
enlever ledit sac à déchets (156) s'il est plein et n'est pas rompu, et remplacer ledit sac à déchets (156) par un nouveau sac à déchets (156).

14. Procédé selon la revendication 13, comprenant en outre l'étape consistant à placer ledit dispositif de scellement (200) portant un code de couleur par-dessus ledit sac à déchets (156) après avoir inséré ledit sac à déchets (156) dans ladite cartouche (106) et coiffer ensuite ledit dispositif de scellement (200) sur ladite cartouche (106), et comprenant également l'étape consistant à enlever ledit dispositif de scellement (200) portant un code de couleur avant de sceller ladite cartouche (106) ou avant d'enlever ledit sac à déchets (156).

15. Procédé pour rejeter de manière sanitaire des déchets en hôpital ou en laboratoire, comprenant les étapes consistant à
fournir un réceptacle à déchets tel que décrit dans la revendication 6 dans lequel ledit bouchon (128) porte un code de couleur ;
insérer ladite cartouche (106) dans ladite ouverture (150) ;
jeter des déchets dans ladite cartouche (106) en plaçant lesdits déchets à travers ledit canal (122) dans ledit bouchon (128) jusque dans la cavité de ladite cartouche (100) ;
sceller ledit canal (122) avec ladite feuille (120) quand la cavité de ladite cartouche (100) est pleine ;
enlever ladite cartouche (106) depuis ladite ouverture ;
remplacer ladite cartouche (106) et ledit bouchon (128) en combinaison par une nouvelle cartouche (106) et un nouveau bouchon (128) en combinaison.
